# EUROPÄISCHE PATENTSCHRIFT

(11) **EP 1 254 229 B1**
(45) Veröffentlichungstag und Bekanntmachung des Hinweises auf die Patenterteilung: **17.11.2004**
(21) Anmeldenummer: 01913553.2
(22) Anmeldetag: 29.01.2001
(51) Int. Cl.: C12N 15/12, C12N 15/53, A61K 48/00, A61K 51/02, A61K 9/127, C12N 15/86, A61P 35/00

(54) **VEKTORKONSTRUKTE ZUR GENTHERAPEUTISCH VERMITTELTEN RADIONUKLID-THERAPIE ENTDIFFERENZIERTER UND MEDULLÄRER SCHILDDRÜSEN-KARZINOME SOWIE NICHT-THYREOIDALER TUMORE UND IHRER METASTASEN**
VECTOR CONSTRUCTS FOR GENE-THERAPY MEDIATED RADIONUCLIDE THERAPY OF UNDIFFERENTIATED AND MEDULLARY THYROID CARCINOMAS AND NON-THYROIDAL TUMOURS AND THE METASTASES THEREOF
STRUCTURES DE VECTEURS DESTINEES A LA THERAPIE PAR RADIONUCLEIDES EN THERAPIE GENETIQUE DE CARCINOMES THYROIDIENS DEDIFFERENCIES ET MEDULLAIRES AINSI QUE DE TUMEURS NON-THYROIDALES ET DE LEURS METASTASES

(30) Priorität: 28.01.2000 DE 10003653
(43) Veröffentlichungstag der Anmeldung: 06.11.2002
(73) Patentinhaber: Medizinische Hochschule Hannover, 30625 Hannover (DE)
(72) Erfinder: PETRICH, Thorsten, 30163 Hannover (DE); PÖTTER, Eyck, 30625 Hannover (DE)
(74) Vertreter: Dehmel, Albrecht, Dr.
(86) Internationale Anmeldenummer: PCT/DE2001/000372
(87) Internationale Veröffentlichungsnummer: WO 2001/055385

(56) Entgegenhaltungen:
- WO-A-97/28175
- MANDELL ROBERT B ET AL: "Radioisotope concentrator gene therapy using the sodium/iodide symporter gene" CANCER RESEARCH,US,AMERICAN ASSOCIATION FOR CANCER RESEARCH, BALTIMORE, MD, Bd. 59, Nr. 3, 1. Februar 1999 (1999-02-01), Seiten 661-668, XP002134372 ISSN: 0008-5472
- DATABASE BIOSIS [Online] BIOSCIENCES INFORMATION SERVICE, PHILADELPHIA, PA, US; 1999 LAZAR VLADIMIR ET AL: "Expression of the Na+/I- symporter gene in human thyroid tumors: A comparison study with other thyroid-specific genes." Database accession no. PREV199900472602 XP002167403 & JOURNAL OF CLINICAL ENDOCRINOLOGY & METABOLISM, Bd. 84, Nr. 9, 1999, Seiten 3228-3234, ISSN: 0021-972X

## Beschreibung

Die vorliegende Erfindung betrifft Vektorkonstrukte, die im Rahmen einer Gentherapie von Tumorerkrankungen mit Hilfe von Liposomen oder Viren als Vektoren eingesetzt werden sollen. In diesen Vektorkonstrukten sind die Gene als cDNA zur Iodination (Iodid-/Radionuklid-Aufnahme) und Iodisation (Iodid-/Radionuklid-Verstoffwechselung) von Thyreozyten sowie Tumor-spezifische Promotoren zur Regulation der Genexpression enthalten.

Varianten solcher Vektorkonstrukte, die das Gen zur Iodination und eine MCS (multiple cloning site) enthalten, sind außerdem über die induzierte Radionuklid-Aufnahme in Zielzellen *in vitro* und *in vivo* (z.B. in Zellkultur, Tiermodell) als Reportergene geeignet. Diese Varianten werden im folgenden als Reportervektoren bezeichnet. Dabei kann einerseits mit Hilfe eines Reportervektors, der eine Expressionskassette zur Vermittlung der Iodid-Aufnahme enthält, die Transfektions-effizienz einer Transfektionsmethode durch alleinige Transfektion und nachfolgende Messung der Radionuklid-Aufnahme in die Zielzelle quantitativ ermittelt und auch lokal über Szintigraphie oder Autoradiographie visualisiert werden. Enthält dieser Reportervektor zusätzlich eine Expressionskassette eines beliebigen anderen Gens, so kann das Expressionsniveau und die Lokalisation des zu untersuchenden Gens über Radionuklidszintigraphie abgeleitet werden.

Andererseits sind Varianten der Reportervektoren, die eine Expressionskassette zur Vermittlung der Radionuklid-Aufnahme ohne Promotor enthalten, zur Bestimmung von Promotor-Aktivitäten beliebiger DNA-Fragmente ebenfalls durch Messung der Radionuklid-Aufnahme geeignet.

Tumorerkrankungen zählen trotz moderner Chirurgie, Strahlentherapie und Chemotherapie nach wie vor zu den häufigsten Todesursachen.

Bei einer seltenen Tumorerkrankung, dem differenzierten Schilddrüsen-Karzinom, gilt die Radioiodtherapie nach vorausgegangener Operation der Schilddrüse als die wichtigste Therapieform. Dabei wird die Eigenschaft der Schilddrüsen-Tumorzellen, Iodid (darunter z.B. auch radioaktives I-131) zu speichern, ausgenutzt, um Tumorreste und Metastasen über eine "innere Strahlentherapie" zu behandeln. Umliegendes normales Gewebe, das kein Iodid speichert, wird dabei geschont.

Die Radioiodtherapie ist bislang außer beim differenzierten Schilddrüsen-Karzinom bei keiner ande-ren Krebsart durchführbar, da nur Schilddrüsen-Karzinome aufgrund spezieller, zur Biosynthese der Schilddrüsenhormone notwendiger Proteine (u.a. Transportproteine, Peroxidasen, Speicherproteine) in der Lage sind, Iodid zu akkumulieren und in gebundener Form längerfristig zu speichern.

### Therapie mit I-131

Nach Angaben der WHO (World Health Organization) waren Krebserkrankungen 1996 die zweithäufigste Todesursache in den industrialisierten Ländern. In vielen Fällen ist mit der klassischen Tumortherapie bestehend aus Operation, Bestrahlung und Chemotherapie keine befriedigende oder keine dauerhafte Tumorremission möglich.

Eine Sonderform der Bestrahlungstherapie stellt die sogenannte Radioiodtherapie (RIT) dar, die auf der Verwendung eines β-Teilchen emittierenden Radioisotops I-131 in Form von Iodid basiert und bislang nur beim differenzierten Schilddrüsen-Karzinom zum Einsatz kommt. Die RIT wird seit ca. 50 Jahren erfolgreich angewendet und basiert auf der Grundlage, dass die Schilddrüse als einziges Organ des menschlichen Körpers Iodid aktiv aufnimmt (Iodination) und zur Synthese der Schilddrü-senhormone (Iodisation) durch Bindung an Tyrosinreste eines intrazellulären Glykoproteins weiter verstoffwechselt. Erst wenn es unter der RIT zu einer Dedifferenzierung der Tumorzellen kommt, kann die Speicherfähigkeit von Iodid und damit die Therapierbarkeit mittels der RIT verloren gehen.

Bleibt die Speicherfähigkeit jedoch über einen längeren Zeitraum erhalten, so kann in mehreren Fraktionen im Abstand von Monaten wiederholt I-131 zur Behandlung von Metastasen, eines Rezidivs oder Resttumors gegeben werden. Limitierender Faktor der RIT ist die Sensibilität des blutbildenden Knochenmarks gegenüber der ionisierenden Strahlung.

Selbst fortgeschrittene Tumorstadien mit ausgedehnter Lungen- oder Knochenmetastasierung sind so in vielen Fällen heilbar. Mit Ausnahme des Knochenmarks nehmen andere Organsysteme keinen deterministischen Schaden, was bei der Chemotherapie, der externen Bestrahlung oder der Operation nicht selten ist.

Neben der Radioiodtherapie mit I-131 bei Schilddrüsen-Karzinomen ist in einzelnen Fällen die Therapie mit I-131-MIBG (Meta-iodo-benzyl-guanidin) bei metastasierenden Phäochromocytomen (Nebennierenmarkstumoren) und Neuroblastomen erfolgreich durchgeführt worden. Mit β-Strahlern radioaktiv markierte Antikörper (AK) sind z.B. bei kolorektalen Karzinomen (anti-CEA-AK) oder bei B-Zell-Lymphomen (anti-CD22-AK) in der Erprobung. Allerdings sind die Heilungschancen bei diesen Tumoren bislang vergleichsweise gering.

### Physiologische Grundlagen

Das in die Schilddrüsenfollikel aufgenommene Iodid wird zunächst oxidiert und in die Tyrosinreste von Thyreoglobulin (TG), einem 660 kD schweren Glykoprotein, eingebaut, wobei intermediär Monoiod- und Diiod-Tyrosinreste (MIT, DIT) entstehen. Etwa 80-90% des Iodids in der Schilddrüse sind im Thyreoglobulin gebunden. Iodid-Oxidation, Iodid-Transfer und die Kopplung von zwei DIT-Resten werden durch die thyreoidale Peroxidase (TPO) unter Verwendung von H₂O₂ katalysiert, wobei TG-gebundenes Thyroxin als Vorstufe des eigentlichen Schilddrüsenhormons (T4) entsteht. TG wird endocytotisch in die Schilddrüsenzellen aufgenommen und anschließend T4 aus TG durch Proteolyse freigesetzt. Thyroidea stimulierendes Hormon (TSH), das in der Hypophyse gebildet wird und auch als rekombinantes, humanes Hormon (rhTSH) kommerziell erhältlich ist, führt über Stimulation des membranständigen TSH-Rezeptors der Thyreozyten über einen second messenger (cAMP) zu einer verstärkten Iodid-Aufnahme in das Cytoplasma und zu einer Beschleunigung der Synthese von TG.

### Molekulare Grundlagen

Während der zugrundeliegende Mechanismus der Iodid-Aufnahme in die Thyreozyten lange Zeit ungeklärt gewesen ist, gelang nach vorläufiger Charakterisierung des Natrium/Iodidsymporters (NIS) als einem membranständigen Transportprotein der Thyreozyten (MG 65 kD) 1996 erstmalig die Isolierung und Klonierung der NIS-cDNA in der Ratten-Schilddrüsenzellinie FRTL-5 (Dai et al. 1996; WO 97/28175) bzw. kurze Zeit später in humanen Thyreozyten (Smanik et al. 1996).

Die NIS-Expression in FRTL-5-Zellen ist TSH-abhängig, was durch Northern-Blot-Analyse nachgewiesen wurde. Die Genexpression beginnt nach 3-6 h und erreicht ein Maximum nach 24h. Mittels Western-Blot-Analyse konnte gezeigt werden, dass die NIS-Protein-Repräsentation an der Zelloberfläche von FRTL-5-Zellen unter TSH-Stimulation nach 36h ansteigt und ein Maximum nach 72 h erreicht - parallel zur Iodid-Transportaktivität.

Über den Vektor pcDNA3 gelang es, in einer Zellkultur anaplastische Schilddrüsen-Zellen mit NIS-cDNA zu transfizieren und eine kurzfristige Iodid-Aufnahme herbeizuführen. Die transfizierten Tumorzellen wurden dann in Ratten implantiert und mit I-131 behandelt. Dabei fand sich eine relativ kurze intrazelluläre Verweildauer des Radioisotops (effektive Halbwertzeit 6h, Spitzenwert nach 90 min). Dies hatte offenbar zur Folge, dass aufgrund der zu geringen Herddosis im Tumor keine Tumor-Reduktion und damit kein Heilungserfolg erzielt wurde. Diese Methode kann somit allenfalls zu diagnostischen Zwecken, nicht aber therapeutisch *in vivo* eingesetzt werden (Shimura et al. 1997).

Ein weiterer möglicher Therapieansatz mit Iod-markierten, organischen Verbindungen (z.B. Antikörper, Oligonukleotide, Rezeptor-Agonisten) würde durch im peripheren Blut vorkommende Deiodasen stark behindert, die eine vorzeitige Zerstörung solcher Tracer verursachen können. Dies führt zum einen zu einer Verkürzung der effektiven Halbwertzeit (HWZ) der Radiopharmaka und zusätzlich zu einer Strahlenexposition anderer Organe (z.B. Leber, Niere, Schilddrüse) durch radioaktive Abbauprodukte.

Andere Tumorarten als differenzierte Schilddrüsen-Karzinome sind bislang mit Radionukliden kurativ nicht behandelbar, da eine ausreichend lange Verweildauer der Radionuklide in den Tumorzellen zur Erreichung einer cytotoxischen Strahlendosis nicht gewährleistet ist.

Zusammenfassend ist derzeit mit Ausnahme des differenzierten Schilddrüsen-Karzinoms keine kurative onkologische Therapie mittels Radionukliden für breite Anwendungen etabliert.

Dementsprechend haben sich die Erfinder die Aufgabe gestellt, eine Möglichkeit zu entwickeln, die Radionuklid-Therapie einer Vielzahl von Tumorarten zugänglich zu machen. Es sollen demnach nicht nur Gene zum Transport von Iodid (WO 97/28175 Mandell et al, Cancer Research, Bd.59, Nr.3, 1999, Seiten 661-668) oder Calcium (WO 98/45443) transfiziert werden, die eine unter Umständen zu kurze effektive HWZ der Radionuklide in den Tumorzellen zur Folge haben, sondern gleichzeitig auch Gene zur Bindung (Organifizierung) der Radionuklide kotransfiziert bzw. im selben Vektor zur Verbesserung der Methode mittransfiziert werden. Des weiteren soll erfindungsgemäß auch mit deutlich toxischeren Strahlern als nur mit I-131 (0,36 MeV; HWZ 8d) therapiert werden. Hierzu wurden von den Erfindern der α-Strahler At-211 (5,8 MeV; HWZ 7,2h) und weitere hochenergetische β-Strahler, z.B. Re-188 (2,1 MeV; HWZ 16,9h) *in vitro* und *in vivo* evaluiert.

Das heißt, auch entdifferenzierte/anaplastische und medulläre Schilddrüsen-Karzinome (Synonym: C-Zell-Karzinome) ohne primäre Radionuklid-Speicherung sowie andere nicht-thyreoidale Tumoren (z.B. der Niere, der Brustdrüse, der Prostata, des Magens, der Lunge, des Knochens, des Pankreas, des Ovars, des Uterus, des Hodens, des Gehirns, endokriner und exokriner Drüsen, der Haut und des Darms), die bislang nuklear-medizinisch nicht behandelbar waren und insbesondere im fortgeschrittenen Stadium kaum wirksam zu beeinflussen sind, werden einer komplementären oder alternativen Therapie mit radioaktivem Iodid oder anderen Radionukliden zugänglich gemacht.

Diese Radionuklide sind insbesondere Astat (At-211) als α-Strahler in verschiedenen negativ geladenen Spezies (z.B. At⁻, AtO⁻, AtO₂⁻, AtO₃⁻, AtO₄⁻), verschiedene negativ geladene Spezies (insbesondere anionische Sauerstoffverbindungen) von β-Strahlern, z.B. von Rhenium (Re-188 und Re-186) oder Yttrium (Y-90), aber auch Auger-Strahler wie Gallium (Ga-67), Indium (In-111) und Iod (I-123).

Durch die erfindungsgemäßen Vektorkonstrukte sollen nicht nur solide Tumore sondern auch Mikrometastasen oder im Blut zirkulierende Tumorzellen über denselben Mechanismus behandelt werden. Die Therapie der Mikrometastasen ist in vielen Fällen entscheidend für die Prognose von Tumorerkrankungen, da durch eine Mikrometastasierung eine generelle Tumorausbreitung im Körper erfolgt. Mikrometastasen, die mit radiologischen Verfahren nur sehr eingeschränkt nachzuweisen sind, können durch lokal begrenzte Therapieverfahren größerer Tumormassen (z.B. Chirurgie, externe Bestrahlung) nicht kurativ therapiert werden.

Die Erfinder haben im Rahmen eines neuen Verfahrens Vektorkonstrukte bereitgestellt, mit deren Hilfe die Eigenschaft zur Aufnahme und Speicherung bestimmter anionischer Radionuklide (Astat, Rhenium, Technetium, Iodid u.a.) in thyreoidale und nicht-thyreoidale Tumorzellen beim Menschen zum Zwecke der Diagnostik und Heilung übertragen werden.

Ein Aspekt der vorliegenden Erfindung betrifft also Vektorkonstrukte, wie sie in den Patentansprüchen beschrieben werden. Ein weiterer Aspekt der vorliegenden Erfindung betrifft die Verwendung dieser Konstrukte zur Herstellung eines Diagnostikums und Therapeutikums für die Diagnose und Therapie von Tumoren, wie sie weiter oben beschrieben worden sind, d.h. insbesondere von Tumor-Erkrankungen wie entdifferenzierte Schilddrüsen-Karzinome, C-Zell-Karzinome sowie nicht-thyreoidale Tumoren und ihre Metastasen. Dabei werden die erfindungsgemäßen Vektorkonstrukte zusammen mit der konventionellen RIT oder anderen Radionuklid-Therapien verwendet, wie es in den Patentansprüchen näher erläutert wird. Besonders geeignet sind die erfindungsgemäßen Konstrukte auch zur Prüfung der lokalen Radionuklid-Speicherung vor der Therapie und zur Detektion bislang nicht bekannter Tumorherde.

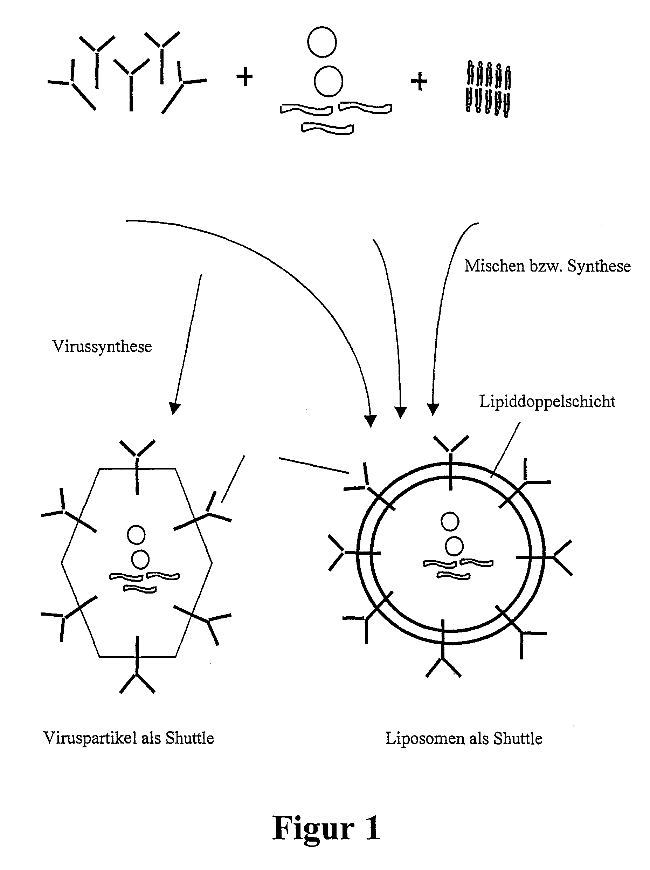

Durch ein selektives "shuttle-system" (tumoraffine Liposomen oder Viren mit entsprechenden Oberflächen-Strukturen) werden die erfindungsgemäßen Vektorkonstrukte in die Tumorzellen geschleust, das NIS- und TPO-Gen sowie ggf. andere relevante Gene zur Schilddrüsenhormon-Synthese (z.B. das TG-Gen (Thyreoglobulin-Gen)) also in diese Zellen (co)transfiziert und mittels z.B. Gewebe spezifischer Promotoren gezielt exprimiert. Im Rahmen des Verfahrens dient dabei das NIS-Gen der zellulären Aufnahme der Radionuklide, die vermittelt durch TPO an zelluläre Substrate oder TG oder TG-Fragmente gebunden werden. Die Bindung an zelluläre Proteine beinhaltet eine Verlänge-rung der zellulären Verweildauer der Radionuklide und erhöht die Strahlendosis (Figur 1, 2 und 3).

Auf diese Weise sollen auch entdifferenzierte und medulläre Schilddrüsen-Karzinome und nicht-thyreoidale Tumoren über eine Radionuklid-Aufnahme und -speicherung behandelbar werden.

Die Applikation der Radionuklide erfolgt im Fall von I-131 in Form von kommerziell erhältlichem Na[I-131]I (Kapseln oder Injektionslösung). Rhenium- und Yttrium-Isotope (wie Re-188 und Y-90) sind ebenfalls kommerziell verfügbar und intravenös applizierbar.

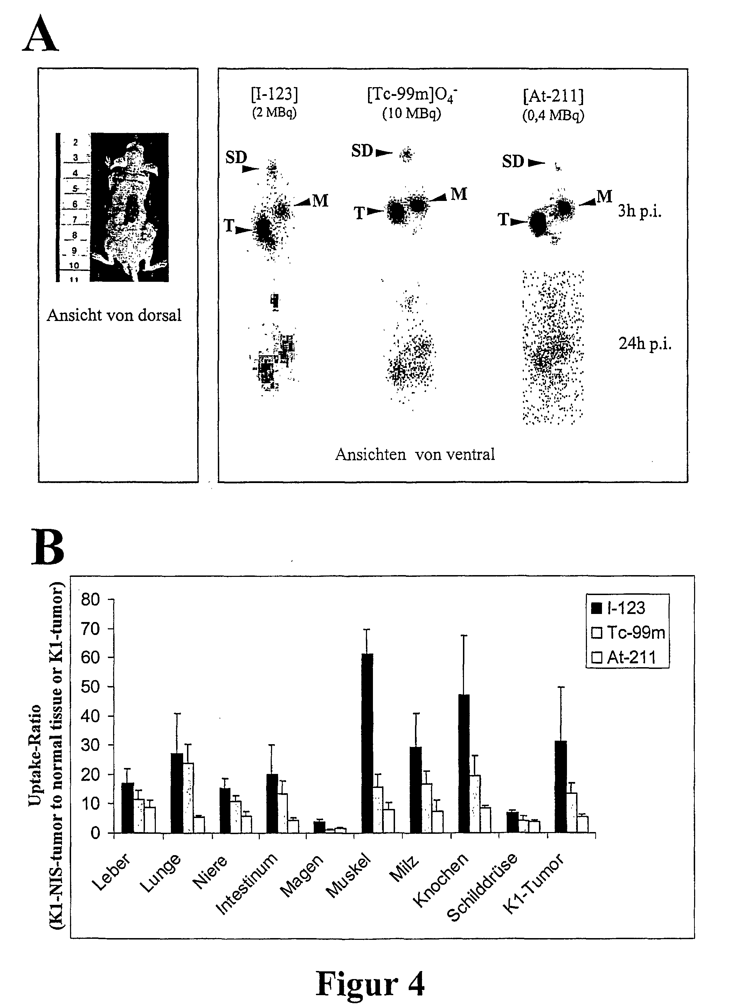

Astat hingegen ist kein natürliches Element. At-211 kann daher im Zyklotron unter Ausnutzung der [²⁰⁹Bi(α,2n)²¹¹At]-Reaktion bei Beschuss von natürlichen Wismut-Targets mit α-Partikeln (24,5 MeV) bei einem Strahlstrom von 6µA erzeugt werden. Das hier verwendete At-211 wurde im Zyklotron (MC35scx, Scanditronix) generiert und durch trockene Destillation bei 650°C aus dem Target ausgeheizt und konzentriert in 0.02 M Na₂SO₃ aufgefangen. Diese At-211-Lösung kann nach entsprechender Verdünnung *in vitro* und *in* vivo (Tiermodell) zur Diagnostik (81 keV γ-Energie zur Szintigraphie) und Therapie verwendet werden (siehe Figur 4).

Figur 1 zeigt schematisch die Übertragung des genetischen Materials. Monoklonale Antikörper (oder andere Zell-spezifische Proteine wie Oberflächen-Antigene und Rezeptor-Liganden) (Y-Form) und Expressionsvektoren (Kreise) mit cDNA und regulatorischen Sequenzen werden mit Lipid-Lösung zu Liposomen als Shuttle-System verarbeitet, wobei die monoklonalen Antikörper oder die anderen Zell-spezifischen Proteine in der Membran des Liposoms verankert, die DNA jedoch in dem Inneren des Liposoms vorliegen. Die monoklonalen Antikörper und die anderen Proteine wie Oberflächen-Antigene und Rezeptor-Liganden dienen der Erkennung der Tumorzellen, sind also bevorzugt, um die selektive Ansteuerung der Zielzellen (also Tumorzellen) durch das Shuttle-System zu ermöglichen. Ein alternatives Shuttle-System ist ein Viruspartikel (Sechseck), bei dem ebenfalls die monoklonalen Antikörper bzw. die anderen Proteine in der Hülle des Virus verankert, die DNA jedoch im Inneren des Virus vorliegen.

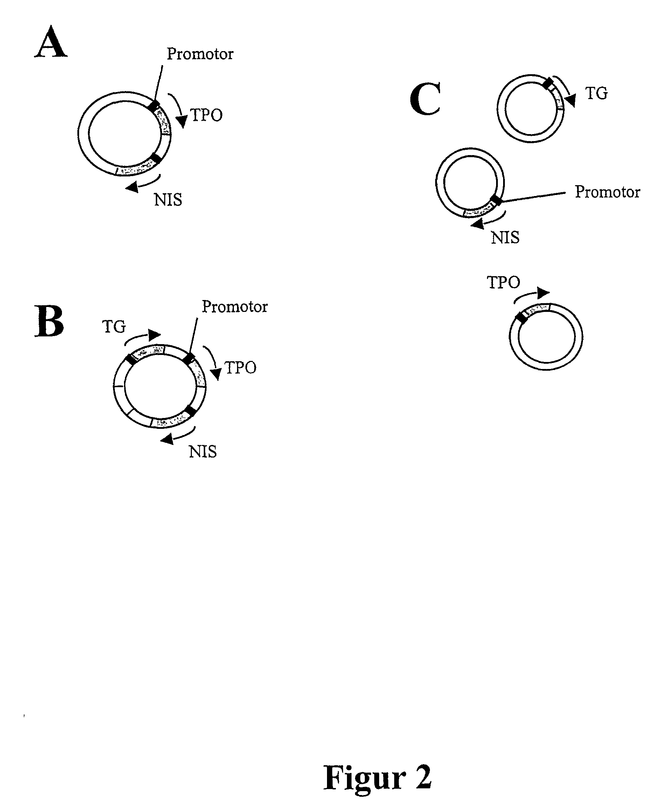

Figur 2 zeigt schematisch die erfindungsgemäßen Vektorkonstrukte: ein Konstrukt (A) mit 2 Genen (und 2 Promotoren), ein Konstrukt (B) mit 3 Genen (und 3 Promotoren) bzw. 3 Konstrukte (C) mit je einem Gen (und einem Promotor). Weitere DNA-Sequenzen (z.B. Polyadenylierungsstellen, regulatorische Sequenzen, Replikationsursprünge oder Gene zur Selektion) sind nicht eingezeichnet, obwohl sie vorhanden sein können oder sogar müssen (wie die regulatorischen Sequenzen).

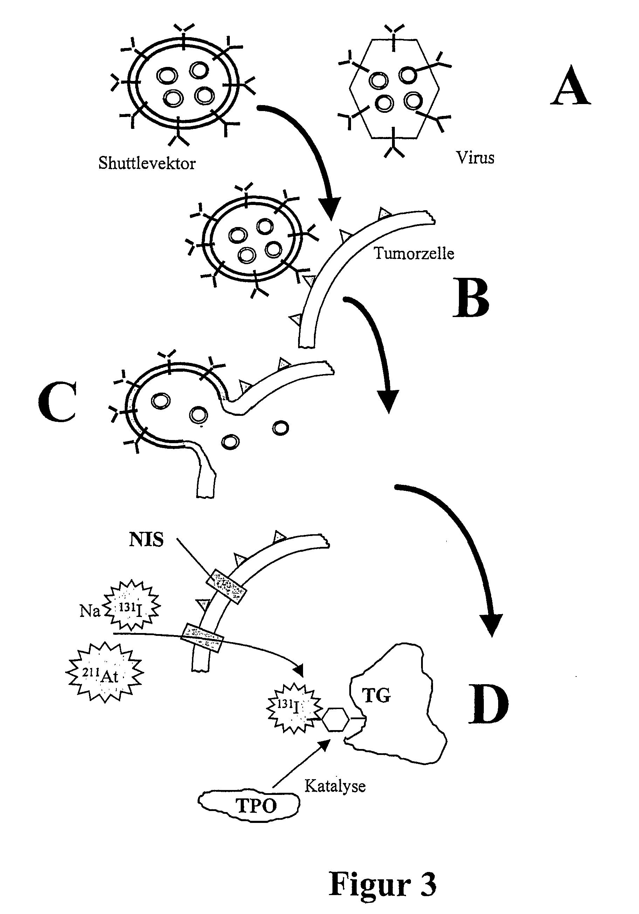

Figur 3 zeigt schematisch den Vorgang der Transfektion. Liposom oder Virus-Partikel (A), die die erfindungsgemäßen Vektorkonstrukte beherbergen, docken aufgrund ihrer Oberflächen-Struktur selektiv bzw. spezifisch an der Membran (mit spezifischen Tumor-Antigenen) von Tumorzellen an (B), verschmelzen mit der Zellmembran (C) und ermöglichen so das Eintreten der Vektorkonstrukte (z.B. über Endocytose) in die Tumorzelle. Dort werden die Gene des/der Vektorkonstrukts/e exprimiert. Als Folge der Expression, insbesondere von NIS, werden die Radionuklide in die Tumorzelle eingeschleust und durch TPO gebunden (D).

Figur 4A zeigt szintigraphische Ergebnisse eines Tiermodells nach subkutaner Injektion von mit hNIS transfizierten Tumorzellen einer Schilddrüsen-Karzinom-Zellinie (K1, ECACC #92030501) in die rechte Flanke einer NMRI-Nacktmaus. Kontralateral (linke Flanke) ist der K1-wt Kontrolltumor zu erkennen. Nach 3-wöchigem Tumorwachstum wurden szintigraphische Aufnahmen von ventral (bauchseitig) mittels Szintigraphie (Gamma-Kamera ZLC370, Siemens) 3 und 24h nach Injektion von 2 MBq I-123, 10 MBq Tc-99m-Pertechnetat und 0,4 MBq At-211 angefertigt (RVL = rechts-ventral-links). Es zeigt sich neben der physiologischen Anreicherung der Tracer in Schilddrüse (SD) und Magen (M) eine signifikante Anreicherung im NIS-transfizierten Tumor und zwar von allen drei Tracern, wobei die Wildtyp-Kontrolltumore (K1-wt) in der linken Flanke nicht zur Darstellung gelangen.

In Figur 4B ist das Verhältnis aus NIS-Tumor-Uptake der drei Tracer (szintigraphisch) und dem Uptake in Normalgewebe bzw. K1-wt-Tumor der drei Tracer dargestellt. Im NIS-Tumor findet sich beispielsweise im Vergleich zu Muskelgewebe ein 60-fach höherer Uptake von I-123, ein 15-fach höherer Uptake von Tc-99m-Pertechnetat und ein 10-fach höherer Uptake von At-211.

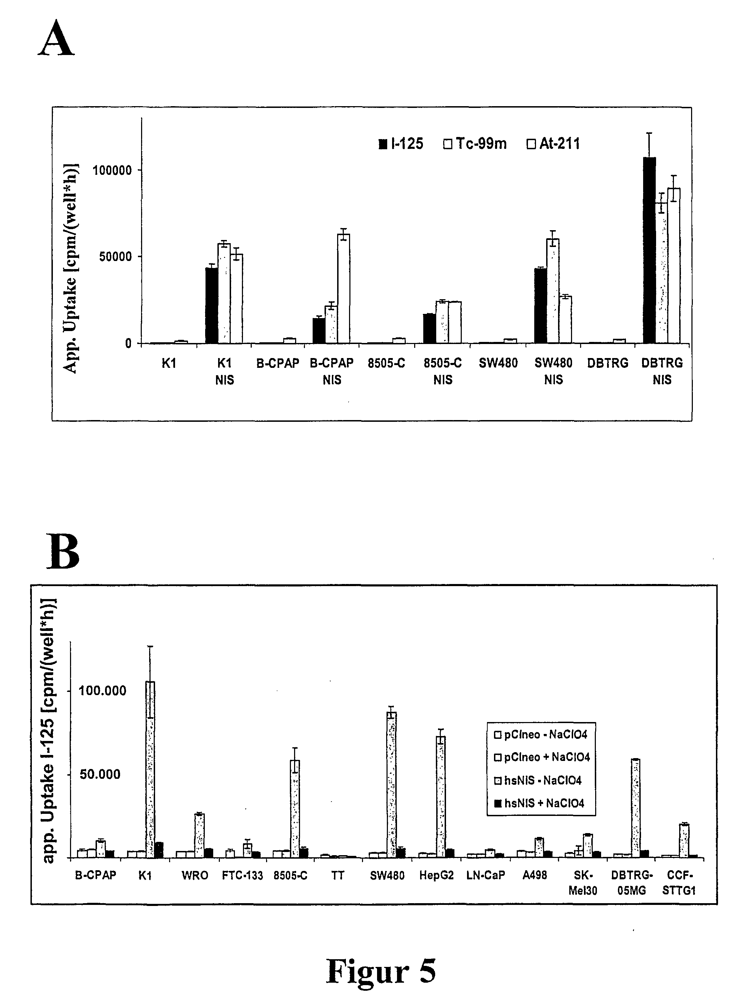

Figur 5A zeigt eigene *in vitro* Ergebnisse der Radionuklid-Aufnahme von I-125, Tc-99m-Pertechnetat und At-211 in stabil mit dem NIS-Gen transfizierten Tumor-Zellinien im Vergleich zu den entsprechenden Kontrollzellen: K1 papilläres Schilddrüsen-Karzinom ECACC #92030501 , B-CPAP papilläres Schilddrüsen-Karzinom DSMZ #ACC273, 8505-C anaplastisches Schilddrüsen-Karzinom DSMZ #ACC219, SW480 Colon-Karzinom DSMZ #ACC313, DBTRG Glioblastom DSMZ #ACC359). Es findet sich eine hochsignifikante Aufnahme aller Nuklide einschließlich des Astats nur in NIS-transfizierten Tumor-Zellinien bei fehlendem Uptake in den Kontroll-Zellen.

In Figur 5B ist die Aufnahme von I-125 zusätzlich in Abhängigkeit einer Perchlorat-Blockade bei transient transfizierten Tumor-Zellinien gezeigt. Neben oben genannten Zellinien wurde ein signifikanter Iodid-Aufnahme in folgenden Zellinien gefunden WRO follikuläres Schilddrüsen-Karzinom ECACC #92030502, HepG2 Hepatozelluläres Karzinom DSMZ #ACC180, LN-Cap Prostata-Karzinom DSMZ #ACC256, A498 Nierenzell-Karzinom DSMZ #ACC55, SK-Me130 Malignes Melanom DSMZ #ACC151, CCF-STTG1 Astrozytom ECACC #90021502, TT Medulläres Schilddrüsen-Karzinom ECACC #92050721). Die Unterschiede der Iodid-Aufnahmeraten in verschieden NIS-transfizierten Zellinien sind durch die variable Transfektionseffizienz der einzelnen Zellinien mit der verwendeten Methode bedingt.

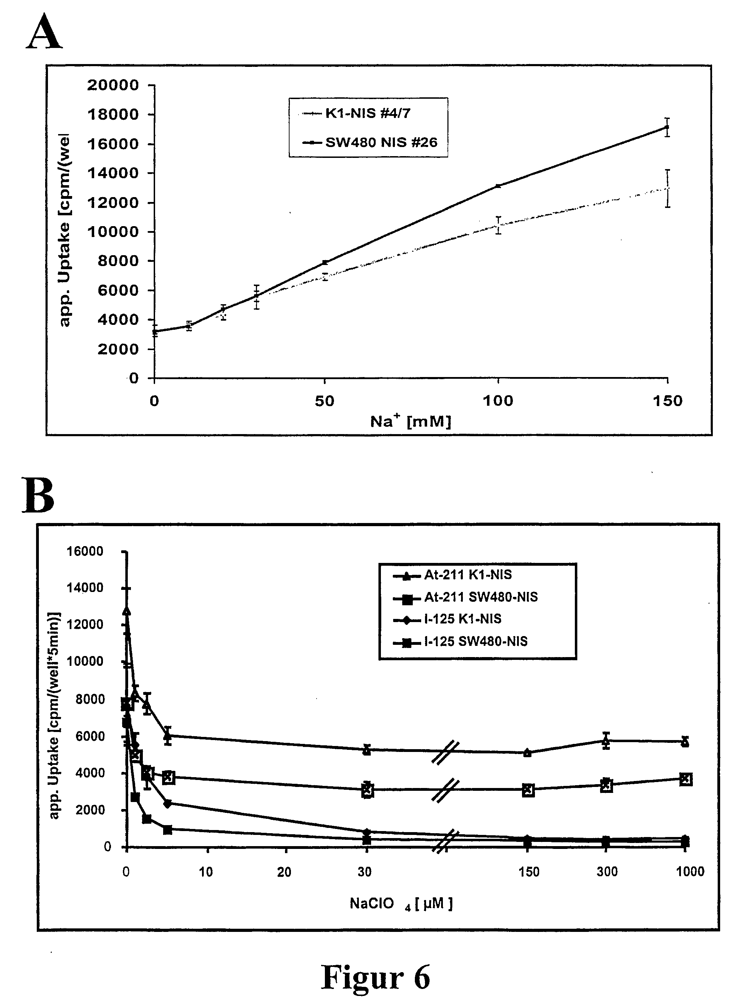

Figur 6A zeigt die Na⁺-Abhängigkeit des At-211-Transports *in vitro* bei stabil transfizierten Tumor-Zellinien (Kl-NIS, SW480-NIS), wobei NaCl durch Cholinchlorid isoosmotisch ersetzt worden ist. Es findet sich eine klare Na⁺-Abhängigkeit des Astat-Transports, was auch beim Iodid bekannt ist. Diese Ergebnisse wie auch die klare Inhibierbarkeit des Astat-Transports durch geringe Perchlorat-Konzentrationen (Figur 6B) bestätigen die These, dass Astat in gleicher Weise wie Iodid über den NIS transportiert wird.

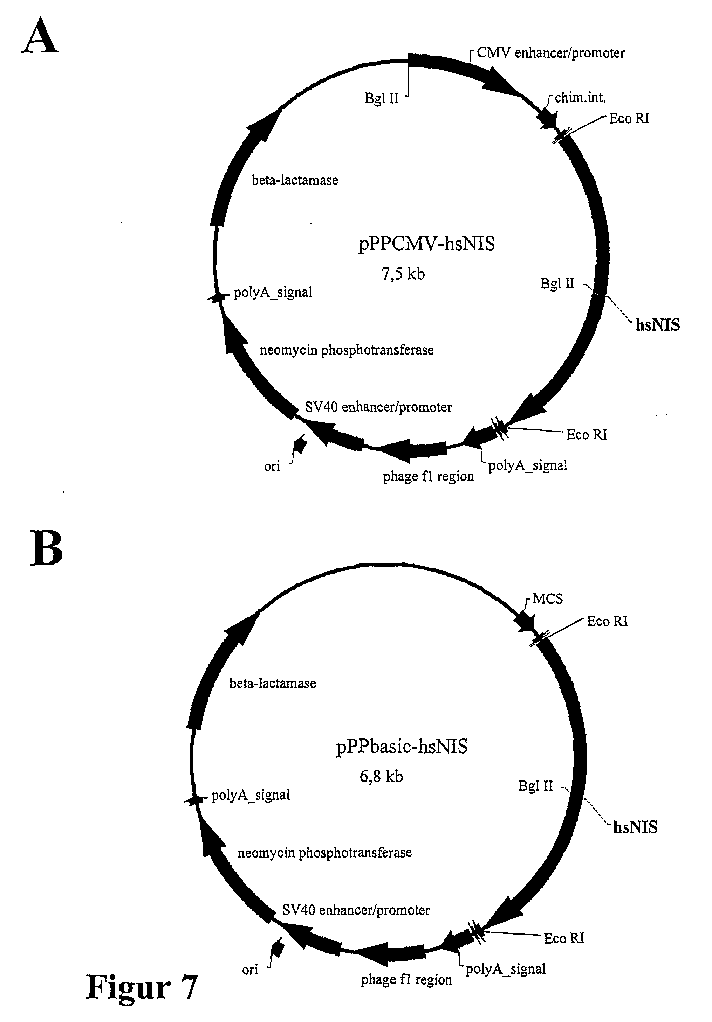

Figur 7A zeigt den Vektor pPPCMV-hcNIS, der sich von dem Vektor pCIneo (Promega GmbH, Mannheim, FRG) ableitet und erfindungsgemäß zur Transfektion des NIS-Gens in verschiedene Tumorzellen sowie zu *in vivo*-Versuchen in Nacktmäusen benutzt wurde. Das humane NIS-Gen (hNIS) steht unter Kontrolle des CMV-Promotors. Der CMV-Promotor kann gegen Gewebe spezifische Promotoren ausgetauscht werden.

Figur 7B zeigt eine Variante des Vektors aus Figur 7A, der zur Überprüfung eines beliebigen in 5'-Position sitzenden Promotors, der in eine MCS kloniert werden kann, geeignet ist.

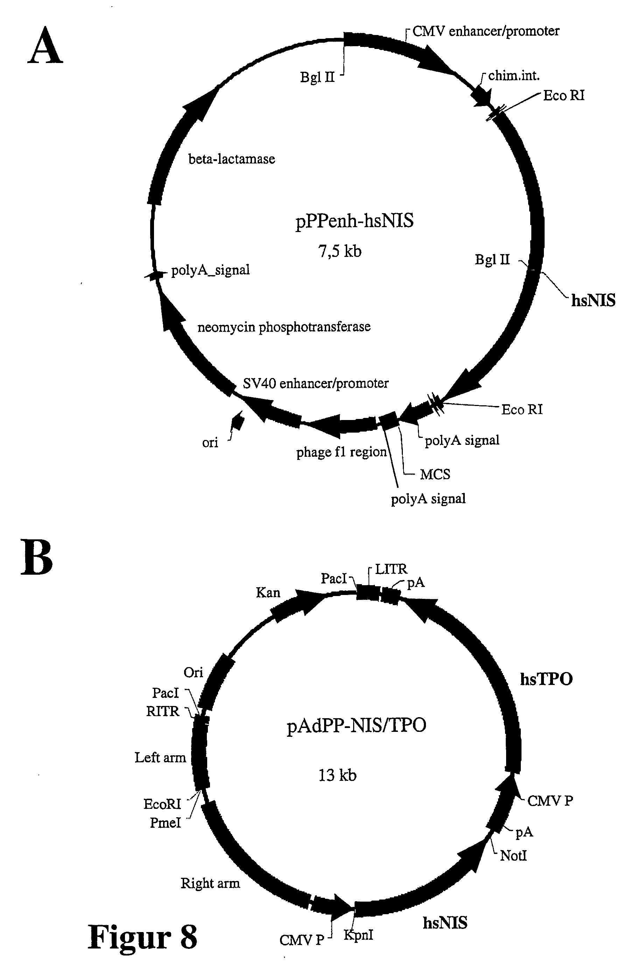

Figur 8A zeigt den Vektor pPPenh-hsNIS, eine weitere Variante des Vektors pPPCMV-NIS, als Reportervektor mit einer in 3'-Position von dem NIS-Gen sitzenden MCS zur Klonierung eines beliebigen Gens. Über die Messung der Iodid-Aufnahme kann *in vivo* oder *in vitro* die Expression eines anderen Gens lokalisiert oder quantifiziert bzw. die Transfektionseffizienz einer Transfektionsmethode untersucht werden.

Figur 8B zeigt den Vektor pAdPP-NIS/TPO, der sich vom Shuttle-Vektor pAdTrack-CMV (He et al. 1997) ableitet und zur Generierung replikationsdefizienter adenoviraler Vektoren geeignet ist, die unter Kontrolle des CMV-Promotors bzw. eines Gewebe spezifischen Promotors gleichzeitig das hNIS-Gen und das humane TPO-Gen in Tumorzellen einbringen und dort exprimieren können.

Gemäß einer weiteren bevorzugten Ausführungsform der vorliegenden Erfindung wird neben dem NIS- und dem TPO-Gen ein drittes Gen (co-)transfiziert: das TG-Gen bzw. ein TG-Genfragment, welches für ein Protein-Teilstück kodiert, das als Iod-Akzeptor dienende Tyrosinreste enthält. Das Thyreoglobulin (TG) dient in der Zelle als Iod- bzw. Iodid-Speicher (Malthiery et al., 1987).

Auf diese Weise wird, wie bereits bei der RIT differenzierter Schilddrüsen-Karzinome praktiziert, eine Verlängerung der effektiven. HWZ und der Bestrahlungsdauer des Tumorzellkerns und damit die Induktion einer therapeutisch wirksamen Dosis (HWZ_{eff} bei der klassischen RIT: 5 Tage) induziert. Erfolgt die (Co-)Transfektion selektiv in die Tumorzellen (z.B. mittels Targeting durch Membran-Epitope oder durch Tumor spezifische Promotoren), kann eine Strahlenschädigung anderer Organe, wie auch bei der klassischen RIT, gering gehalten werden (Strahlenbelastung des Gesamtorganismus: ca. 300 Milli-Sievert [mSv] bei einer Standardaktivität bei der RIT von ca. 11,1 Giga-Bequerel [GBq]). Eine Knochenmarksaplasie oder Knochenmarksdepression kann vermieden werden, wenn die Teilkörper-Dosis des Knochenmarks 2 Gray [Gy] nicht überschreitet. Entspre-chende Dosis-Messungen sind unter der Therapie möglich und gehören zum Stand der Technik.

Die Übertragung des genetischen Materials in die Tumorzellen erfolgt durch Shuttle-Vektoren. Diese Shuttle-Vektoren sind zum einen Liposomen, die membranständige monoklonale Antikörper (AK), Liganden oder Proteine zur Erkennung bestimmter Tumorzell-Oberflächen-Strukturen aufweisen. Diese in die Liposomen-Membran integrierten Proteine oder AK haben die Funktion, eine erste Selektion im Rahmen des Gewebe spezifischen Targetings vorzunehmen, um so die zu transfizierenden Gene mit höherer Wahrscheinlichkeit in Tumorzellen als in normale Zellen zu transportieren. Alternativ können auch Tumor spezifische, präparierte, replikationsdefiziente Viren (z.B. Adenoviren, Lentiviren, Retroviren, andere DNA-Viren) mit entsprechenden Epitopen Verwendung finden: Adenovirale Vektoren bewirken eine transiente Transfektion, da die DNA im Unterschied zu Retroviren nicht in das Genom integriert und entsprechend nach einigen Zellteilungen verloren geht. Adenoviren sind im Gegensatz zu Retroviren bei der Infektion und Genexpression nicht auf proliferierende Zellen angewiesen, was von Vorteil bei langsam wachsenden oder ruhenden Tumoren ist. Retroviren sind nur bei sich teilenden Tumorzellen verwendbar - diese sind dann dauerhaft transfiziert. Diese Gründe sprechen für einen bevorzugten Einsatz von human- oder tier-pathogenen (z.B. Schaf) adenoviralen Vektoren. Zudem ist es möglich, Adenoviren so zu modifizieren, dass sie bestimmte Tumor typische Bindungsstellen erkennen. Adenoviren verfügen über sogenannte Fiber-Proteine mit endständigen "Knöpfchen" (Trimer, L-Bereich IV "late Expression", 62 kD), mit deren Hilfe die Viren an membranständige Rezeptoren (CAR, Coxsackie and Adenovirus Receptor) ihrer Zielzellen binden und dann über Endocytose ins Cytoplasma gelangen [Bergelson et al. 1997]. Die Struktur dieser Knöpfchen wird über die Virus-DNA (L-Bereich) kodiert und kann durch Änderung der zugrunde liegenden Sequenz modifiziert. Wenn man in die für die Knöpfchen kodierende Sequenz die entsprechende Sequenz aus monoklonalen AK gegen bestimmte Membran-Epitope (z.B. PSMA) kloniert bzw. diese dadurch ersetzt, können die Viren vermehrt Zellen befallen, die PSMA (Prostata spezifisches Membran-Antigen) exprimieren (z.B. Prostata-Karzinom). Auf diese Weise gelingt eine Vorselektion der Tumorzellen in Bezug auf eine spätere Therapie mit Radionukliden. Da die Knöpfchen in ihrer ursprünglichen Struktur immunogen wirken, kann eine Strukturänderung über Modifizierung des Virusgenoms in Richtung menschlicher Tumorzell-Epitope die Immunogenität der Fiberproteine senken und somit eine mehrfache Anwendung am Menschen und eine Senkung des Allergie-Risikos ermöglichen.

Die Erzeugung solcher Viruspartikel oder Liposomen ist bereits beschrieben worden und Stand der Technik (Strauss et al., 1997; Tarhovsky et al., 1998; Anderson, 1998; Martin et al., 1999; Kurane et al., 1998). Eine weitere Möglichkeit, die therapeutischen Gene in Tumorzellen zu übertragen, ist die Applikation von Virus produzierenden Zellen (z.B. psi 2-BAG-Verpackungszellinie) direkt in den Tumor, in benachbartes Gewebe oder andemorts, z.B. in die Muskulatur. Die im Körper von den implantierten Zellen ("producer cells") produzierten Viruspartikel befinden sich dann direkt im Tumor oder in Tumomähe und können die Tumorzellen transformieren (Short et al. 1990). Setzt man bei dieser Applikationsart Tumor spezifische Promotoren in den Vektorkonstrukten oder Tumor spezifische Oberflächen-Strukturen der Virushüllen (z.B. Fiber-Proteine) ein, können die Virus produzierenden Zellen auch entfernt vom Tumor im Körper eingesetzt werden, da die generierten Viruspartikel nur Tumorzellen mit entsprechenden Epitopen befallen bzw. die therapeutischen Gene nur Tumor spezifisch exprimiert werden.

Als Tumormembran-Epitope kommen beispielsweise in Frage:
- Mamma-Karzinom: erbB2 (Slamon, 1987)
- Ovarial-Karzinom: CA125, HMFG1 und HMFG2 (Metcalf et al. 1998),
- Prostata-Karzinom: PSMA (Murphy et al. 1998),
- Malignes Melanom: Melan-A/MART-1-AG (Schneider et al. 1998).

Die eigentliche Tumor-Spezifizität soll durch eine gezielte Regulation der Genexpression nur in den Tumorzellen erfolgen. Deshalb sind vor die zu transfizierenden Gene in den Vektor-Konstrukten Tumor spezifische Promotoren geschaltet, die eine Expression der Proteine (NIS, TPO, TG, nachfolgend auch als therapeutische Proteine bezeichnet) nur in bestimmten Geweben - je nach Art der Tumoren - zulassen. Als geeignete Promotoren kommen Promotoren von Tumor spezifischen Proteinen in Frage. Diese Proteine können Tumor-Marker (z.B. TG, CEA, Calcitonin, AFP, CA-19-9, PSA), Rezeptoren (z.B. SMS-Rezeptor), Membran-Proteine (z.B. PSMA), Enzyme (z.B. NSE), Hormone (Prolaktin, HCG) oder andere Peptide sein, die in hohem Maße von den Tumorzellen und nur von wenigen Körperzellen produziert werden. Somit wird unter Kontrolle bestimmter Promotoren eine Tumor-spezifische Überexpression der zu transfizierenden Gene gewährleistet und die Radionuklide nur in bestimmten Tumorzellen, nicht aber im normalen Gewebe (auch wenn dieses ebenfalls durch Viren oder Liposomen transfiziert werden sollte), gespeichert. Tumor-spezifische Promotoren sind bekannt und sequenziert und kommen für diese Art der Therapie in Betracht. Die gezielte Expression von Genen unter Kontrolle bestimmter Promotoren ist Stand der Technik. Folgende Promotoren kommen beispielsweise bei den entsprechenden Tumorerkrankungen zur Therapie in Betracht:
erbB2-, Ca-15-3-Promotor: Mamma-Karzinom
Calcitonin-Promotor: medulläres Schilddrüsen-Karzinom
CEA-Promotor: Magen-, Darmtumor, anaplastisches Schilddrüsen-Karzinom, Bronchial-Karzinom, Ovarial-Karzinom
TG-Promotor: papilläre und follikuläre Schilddrüsen-Karzinome
NSE-Promotor: kleinzelliges Bronchialkarzinom
PSA-, Saure-Phosphatase-Promotor: Prostata-Karzinom
SMS-Rezeptor-Promotor: Nierenzell-Karzinom, medulläres Schilddrüsen-Karzinom, Karzinoide, Hypophysen-Tumor
AFP-Promotor: Uterus-Karzinom, Leber-, Ovarial- und Hoden-Tumor
HCG-, LDH-Promotor: Ovarial- und Hoden-Tumor
SCC-Promotor: Zervix-Karzinom, Lungentumor-epithelial
CA-19-9-, Ca-50-Promotor: Kolon-, Pankreas-Karzinom
Ca-125-Promotor: Ovarial-Tumoren, epitheliale Tumoren
ACTH-Promotor: Bronchial-, Mamma-, Pankreas-, Magen-Karzinom
Prolaktin-Promotor: Hypophysen-Tumoren

In den Vektorkonstrukten müssen die therapeutischen Gene mit den entsprechenden Promotoren je nach Tumorerkrankung individuell kombiniert werden. Die Vektorkonstrukte werden gemäß einer bevorzugten Ausführungsform in Liposomen verpackt, in deren Membranen Antikörper oder Proteine gegen Tumorzell-Oberflächenstrukturen wie PSMA-AK, SMS-Analoga, oder erbB3-AK integriert vorliegen. Die Transfektion der therapeutischen Gene erfolgt vorzugsweise mit einer Anzahl von zirkulären Vektoren, die der Anzahl der zu transfizierenden therapeutischen Gene entspricht. Dabei weisen die zirkulären Vektoren jeweils (i) eine Promotor-Region (z.B. SV40-P_{E}, CMV-Promotor oder Gewebe spezifischer Promotor wie PSA- oder CEA-Promotor, (ii) einen Säugetierzell-wirksamen Replikationsursprung (z.B. Repori_{BAk}./Repori_{Mam}.), (üi) ein Antibiotikum-Resistenzgen (z.B. amp^{r}, neo^{r}), (iv) ein Polyadenylierungssignal (z.B. Basenfolge: AATAAA) und (v) cDNA für eines der therapeutischen Proteine auf.

Gemäß einer anderen bevorzugten Ausführungsform (Figur 7A, Beispiel) werden die Vektorkonstrukte ebenfalls in Liposomen verpackt, in deren Membranen Antikörper oder Proteine gegen Tumorzell-Oberflächenstrukturen integriert vorliegen. Die Transfektion der zwei bzw. drei therapeutischen Gene (NIS, TPO, TG) erfolgt nun jedoch mit Hilfe von einem ringförmigen Vektor, der jeweils neben den o.g. DNA-Segmenten (i) bis (iv) die cDNAs von zwei bzw. von drei therapeutischen Genen enthalten (z.B. Vektor a) mit NIS- und TPO-cDNA und Vektor b) mit NIS-, TPO- und TG-cDNA).

Eine andere erfindungsgemäß bevorzugte Ausführungsform (Figur 8b, Beispiel) sieht als Shuttle-Vektoren anstelle der Liposomen virale Vektoren vor. Eine bevorzugte Variante ist die Integration des NIS- und des TPO-Gens mit entsprechender Tumor spezifischer Promotor-Region oder einem konstitutiven Promotor (z.B. von CMV, SV40) in replikationsdefiziente adenovirale Genome (z.B. pShuttle/Ad-easy-Kit, Quantum), deren Oberflächen-Antigene mit Tumor spezifisch veränderten Oberflächen-Proteinen (z.B. gegen PSMA-Antigen, SMS-Rezeptor, erbB3-Antigen) ergänzt werden können. Genauso können aber auch alle drei Gene (NIS, TPO, TG) gleichzeitig in einem oder getrennt in drei verschiedenen viralen Vektoren mit entsprechender Tumor spezifischer Promotor-Region oder entsprechend in verschiedene retrovirale Vektoren mit bestimmten Tumor spezifischen Oberflächen-Proteinen (z.B. gegen PSMA-Antigen, SMS-Rezeptor, erbB3-Antigen) integriert sein.

Als Vektorkonstrukte kommen ringförmig geschlossene, nicht-restriktive Expressionsvektoren, wie z.B. Simian Virus 40 (SV40) oder ein hiervon abgeleiteter pSV2-Typ, aber auch Derivate von Adenovirus-, Retrovirus- oder Herpesvirus-Genomen in Frage, die eine möglichst hohe Transkriptionsrate im eukaryotischen Zellkern erreichen. Ziel ist die Expression der therapeutischen Gene und die Repräsentation der entsprechenden "neuen" thyreoidalen Proteine an der Zellmembran bzw. im Cytoplasma der Tumorzellen. Die Herstellung der Vektor-Konstrukte und die Klonierung in Virus-Genome ist Stand der Technik (siehe auch Sambrook et al.: *Molecular Cloning*).

Die therapeutische Applikation der Liposomen oder human- oder tier-pathogenen Viren bzw. diese Viren produzierenden Zellen erfolgt intravenös, intraperitoneal, intrathekal, intracraniell, intrathorakal, endobronchial, endolymphatisch, intraarteriell und intratumoral (im Fall der Applikation der Zellen: bevorzugt intratumoral). Bei intravenöser, systemischer Gabe sind in den Vektorkonstrukten bevorzugt Tumor spezifische Promotoren zu verwenden. Bei direkter Applikation im Rahmen einer lokalen Therapie in den Tumor (z.B. intratumoral oder in eine den Tumor versorgende Arterie) können auch nicht Tumor spezifische konstitutive Promotoren (z.B. CMV- oder SV40-Promotor) verwendet werden.

Vor Beginn einer Therapie sollte auf das Vorhandensein entsprechend hoher Tumor-Markerwerte oder einer entsprechend hohen Expression der Rezeptoren oder membranständigen Proteine oder sonstiger Tumor spezifischer Proteine geachtet werden, z.B. durch Blutentnahme oder Biopsie aus Tumorgewebe.

### Klonierung

Die Synthese der cDNAs (der therapeutischen Gene) gehört zum Stand der Technik. Das gilt gleichermaßen für die Herstellung der zirkulären (oder linearen) Vektorkonstrukte mit den entsprechenden regulatorischen Elementen und Restriktionsschnittstellen zum Einbau der cDNAs. Bevorzugter Weise verfügt jede cDNA eines Vektorkonstrukts über eine eigene, effiziente Promotor-Region, z.B. die des CMV (immediate early Promotor/Enhancer) oder die des SV40-Virus (SV40-P_{E}), die des Rous-Sarkom-Virus (RSV-LTR) oder die des Thymidinkinase-Gens des Herpes-Simplex-Virus (HSV-TK). Auch können mehrere therapeutische Gene von einem gemeinsamen Promotor aus reguliert werden. Dabei müssen wenigstens zwei der therapeutischen Gene auf einem einzigen Vektormolekül lokalisiert sein.

Die cDNAs der therapeutischen Gene werden aus humaner Thyreozyten-mRNA nach reverser Transkription durch nachfolgende PCR mir Gen spezifischen Primern amplifiziert und erhaltene Fragmente in einem Klonierungsvektor (z.B. pCRblunt, Invitrogen) in E. coli (K12-Derivat) subkloniert. Dabei werden überlappende cDNA-Segmente der einzelnen Gene verwendet, die es aufgrund gemeinsamer Restriktionsenzym-Schnittstellen erlauben, "full length"-cDNA's zu erzeugen. Die cDNAs werden in einen Vektor zur konstitutiven Expression in Säugetierzellen kloniert (z.B. in pCIneo unter Kontrolle des CMV-Promotors, Promega). Die Expressionsvektoren mit integrierter cDNA (Figur 7A, Beispiel) werden für transiente Transfektionen verschiedener Tumor-Zellinien verwendet (Fugen6, Roche), sowie zur Erzeugung von stabil transfizierten Zellinien durch Antibiotika-Selektion (z.B. G418 für pCIneo). Radionuklid-Aufnahme-Messungen [I-125], [At-211], [Tc-99m], [Re-188]) an Zellkulturen (Figur 5 und 6) erfolgen entsprechend der Methode nach Weiss et al. 1984. Zur Etablierung eines Tiermodells werden stabil transfizierte Tumor-Zellinien bzw. entsprechende Kontrollzellen in Nacktmäuse (nu/nu) NMRI subkutan injiziert. Nach 3-4-wöchigem Tumorwachstum und Applikation verschiedener Radionuklide (u.a. [I-123], [At-211], [Tc-99m]) werden sequenzielle Szintigramme mit einer Gamma-Kamera angefertigt und Organverteilungen über region-ofinterest (ROI)-Analysen bestimmt (Figur 4).

Die Ergebnisse der *in vitro* und *in vivo* (Tiermodell) Versuche zeigen, dass durch die beschriebene Transfektion der therapeutischen Gene eine signifikant höhere Radionuklid-Aufnahme in Tumorzellen als im normalen Gewebe oder in den Kontroll-Tumoren induzierbar ist. Dies gilt insbesondere auch für Astat, was bislang noch nicht publiziert wurde. Die bei der Transfektion mit Hilfe der erfindungsgemäßen Vektoren exprimierten Proteine werden im Western Blot mit spezifischen Antikörpern nachgewiesen. Eine Organifizierung der Radionuklide mit Hilfe der Vektoren wird durch vergleichende Studien mit NIS-Transfektanten und Kontroll-Zellen durch Messung der Ethanol-Pellets nachgewiesen. Die Organifizierung der Radionuklide, die entscheidend ist für die Verlängerung der effektiven HWZ, gelingt also durch den zusätzlichen Einsatz von TPO. In Efflux-Versuchen werden durch Mediumwechsel der Zellkultur-Schalen nach vorheriger Inkubation mit verschiedenen Radionukliden nur sehr kurze HWZ bei der Transfektion mit dem NIS-Gen allein festgestellt (HWZ: 5 min). Die Cotransfektion von NIS und TPO bzw. von NIS, TPO und TG bewirkt hingegen eine signifikante Verlängerung der Radionuklid-Bindung um den Faktor 7-10 *in vitro* und damit eine Erhöhung der Strahlendosis in den transfizierten Tumorzellen. Apoptose-Raten werden in clonogenen Assays verglichen nach Transfektion mit NIS allein gegenüber einer Cotransfektion mit NIS und TPO nach Inkubation mit α-/β-emittierenden Radionukliden. Dabei zeigt sich eine Steigerung der Apoptose-Rate bei Cotransfektion beider Gene um den Faktor 20-30 gegenüber einer NIS-Transfektion sowohl bei Tumorzellen aus der Schilddrüse wie auch aus anderen Geweben. Neben Western Blot Analysen zum Nachweis der Proteine (NIS, TPO, TG) wird die Enzym-(Peroxidase)-Aktivität von TPO in parallelen Ansätzen im Guaiacol-Assay bestimmt.

Vektor-Varianten zur Ermittlung der Transfektionseffizienz, der Promotor-Aktivität und der visuellen Darstellung einer Transfektion werden ermittelt durch Cotransfektion von LN-CaP-Zellen (Prostata-Karzinom, DSMZ #ACC-256) mit den Vektoren pPPbasic-hsNIS (CMV- und PSA-Promotor vs. ohne Promotor) und pPPenh-hsNIS mit pSV-βGal und pEGFP (Promega).

Körperzellen, die die Vektorkonstrukte bei einer Therapie eventuell "en passant" aufnehmen, besitzen nicht die relevanten Transkriptionsfaktoren, die zum Anschalten der Promotor abhängigen Expression notwendig sind. Deshalb können diese die transfizierten Gene nicht exprimieren, keine Radioisotope speichern und damit keinen wesentlichen Schaden nehmen. Somit kann durch die individuelle Wahl eines geeigneten Promotors eine zusätzliche Sicherheit der Therapie gewährleistet werden.

Eine Therapie einzelner, nicht erfolgreich transfizierter Tumorzellen wird auch durch "Nachbarschaftsbestrahlung" aus transfizierten Tumorzellen erreicht. Denn Elektronen von β-Strahlem der Energie 0,61 MeV (I-131) legen in Wasser/Gewebe eine Strecke von 2 mm (im Mittel 1 mm) zurück, was ca. 40 Zellschichten entspricht. Deutlich längere Strecken im Gewebe sind für Re-188 und Y-90 bekannt. α-Partikel, die beim Zerfall von At-211 entstehen, haben dem gegenüber eine sehr viel höhere Energie (> 5 MeV) und aufgrund ihrer großen Masse eine sehr viel höhere Ionisationsdichte, was auch ihre stärkere Toxizität erklärt. Man nimmt an, dass nur ein α-Zerfall im Inneren einer Zelle ausreicht, um diese abzutöten. Dabei spielen neben der Ionisation auch Rückstoßkräfte eine Rolle, die beim Zerfall auftreten und die zu erheblichen Zerstörungen der intrazellulären Matrix führen. Aus diesem Grund ist das Ziel der vorliegenden Erfindung auch, insbesondere At-211 in die Tumorzellen zu befördern und das Radionuklid dort, wo es am meisten schadet, zu fixieren.

Vor einer Transfektion wird der Rezeptorstatus bzw. der Status bestimmter Membran-Epitope des Tumors geprüft, um eine effiziente Einschleusung der therapeutischen Gene sicherzustellen. Dies kann durch eine Immuncytologie, Immunhistologie oder Immunszintigraphie erfolgen.

Außerdem ist eine Blockierung der Iodid- bzw. Radionuklid-Aufnahme der Schilddrüse mit kurzzeitiger, hoch dosierter Gabe von Schilddrüsen-Hormonen (z.B. 1 mg/d L-Thyroxin über 3 Tage) zur Suppression der hypophysären TSH-Produktion erforderlich, um zu verhindern, dass größere Mengen der Radionuklide von der Schilddrüse abgefangen werden. In extremen Fällen einer Hyperthyreose muß ggf. vor einer Tumorbehandlung eine Thyreoidektomie erfolgen, weil Radioiod oder andere Radionuklide physiologischer Weise bevorzugt durch die Schilddrüse aufgenommen werden und somit dem Einbau in die Tumorzellen nicht mehr zur Verfügung stehen. In solchen Fällen kann posttherapeutisch (nach Thyreoidektomie) eine Substitution mit Schilddrüsen-Hormon durchgeführt werden.

Mit Beginn der eigentlichen Behandlung, wie sie hier beschrieben ist, werden Shuttle-Vektoren mit den therapeutischen Genen (NIS, TPO, TG) *in vitro* bestückt bzw. virale Vektoren wie oben beschrieben generiert und in einer Träger-Lösung z.B. intravenös appliziert (Figur 1).

Nach einem Intervall von etwa 2-4 Tagen sind die therapeutischen Gene in die Tumorzellen eingeschleust. Die Synthese der therapeutischen Proteine (NIS, TPO, TG) erfolgt nach Transkription der cDNA im Zellkern durch Translation der mRNA im Tumor-Cytoplasma. Zu diesem Zeitpunkt sollte daher eine Radionuklid-Ganzkörperszintigraphie (z.B. Testuntersuchung mit einer geringen Aktivität von ca. 50-100 MBq I-131) durchgeführt werden, um die erworbene Speicherfähigkeit intratumoral zu überprüfen. In Abhängigkeit von der erworbenen Speicherfähigkeit des Tumorgewebes wird eine hoch dosierte Radionuklid-Therapie (mit z.B. 4-12 GBq I-131) stationär angeschlossen.

Aus Strahlenschutz-Gründen muß die Therapie auf einer nuklear-medizinischen Station, die für den Umgang mit offenen Radionukliden eingerichtet ist (Abschirmung, Abklinganlage), durchgeführt werden. Die Kombination aus Transfektion und Radionuklid-Therapie kann bei guter Toleranz mehrmals wiederholt werden. Sollte sich eine Immunisierung gegen die Viren oder Liposomen ergeben, muß das "Transportvehikel" gewechselt oder eine kurzzeitige immun-suppressive Therapie eingeleitet werden. Eine vorsichtige Immun-Suppression trotz Infektion mit Viren ist möglich, da die injizierten Viren replikationsdefizient sind und sich im Körper nicht vermehren können. Ähnlich wie bei der klassischen RIT sind unter Strahlenschutz-Gesichtspunkten maximal 4-5 Fraktionen pro Jahr sinnvoll. Bevorzugt ist jedoch eine nur transiente Transfektion (3-4 Wochen) mit Hilfe von adenoviralen Vektoren, da die Expression der therapeutischen Gene nur kurzzeitig für die Radionuklid-Therapie erforderlich ist und nicht dauerhaft erfolgen muß.

Durch die erfindungsgemäßen Vektorkonstrukte wird lediglich eine transiente Transkription und Überexpression ermöglicht (Ausnahme: bei der Verwendung von Retroviren als Shuttle-System), die unter Umständen nur über wenige Zellteilungen erhalten bleibt, aber für den Zweck der anschließenden Radionuklid-Therapie mit vorübergehende Speicherung und anschließender Apoptose der Tumorzellen ausreicht.

Die erfindungsgemäße Verwendung der oben ausführlich beschriebenen Vektorkonstrukte, die die für die Radionuklid-Aufnahme und Organifizierung verantwortlichen Gene enthalten, hat folgende entscheidende Vorteile:
- Entdifferenzierte und medulläre Schilddrüsen- sowie C-Zell-Karzinome erhalten die Eigenschaft, Radionuklide aufzunehmen und zu speichern.
- Auch andere Tumorarten als die differenzierten Schilddrüsen-Karzinome können durch eine gentechnische Veränderung längerfristig Radionuklide speichern und werden so nuklear-medizinisch behandelbar.
- Die Speicherung von Radionukliden ist selektiv nur in transfizierten Zellen möglich, alle anderen Organe speichern nicht und werden so geschont.
- Eine Schädigung umliegenden Gewebes entfällt, da die α-/β-Strahlung keine große Gewebestrecke zurücklegt und im wesentlichen die DNA der speichernden Tumorzellen bzw. der unmittelbar benachbarten Zellen zerstört. Somit ist die Behandlungsdauer im Gegensatz zu der bei externer Bestrahlung nicht limitiert (ausgenommen Knochenmark).
- Werden nur einige der Tumorzellen erfolgreich transfiziert, können angrenzende nicht transfizierte Tumorzellen durch eine "Nachbarschaftsbestrahlung" ebenfalls geschädigt werden. Dabei können Radionuklide zusätzlich über Zellporen (gap-junctions) in angrenzende, nicht transfizierte Tumorzellen gelangen und diese so schädigen.
- Die Strahlendosis intratumoral ist bei der Radionuklid-Therapie bedeutend höher (>300Gy) als bei der externen Bestrahlung (ca. 40-60Gy). Somit ist auch die Toxizität und der therapeutische Effekt im Tumor bei geringeren zu erwartenden lokalen Nebenwirkungen stärker.
- Selbst wenn nur eine vorübergehende Radionuklid-Speicherung durch transiente Genexpression stattfindet, reicht dieser Effekt aus, um eine Therapie durchzuführen.
- Da vor Beginn der Radionuklid-Therapie der Rezeptor-/Membranstatus des Tumors bestimmt wird, und Liposomen bzw. Viren dementsprechend präpariert werden können, ist das Risiko für Nebenwirkungen gering.
- Durch die Wahl vorgeschalteter, das heisst in 5-Position lokalisierter Tumor spezifischer Promotoren je nach Tumormarker-Nachweis oder Proteinsynthese des Tumors zur Regulation der Genexpression kann eine hohe Selektivität der Tumorbehandlung erreicht werden.
- Die transfizierten Tumorzellen sind zum Staging (Ausbreitungsdiagnostik) oder zur Verlaufskontrolle nach Therapie über eine Szintigraphie mit Gammakameras (oder in größerer Genauigkeit mittels Positronen-Emissionstomographie mit dem Positronen-Emitter [I-124]) detektierbar.
- Limitierend bei der Radionuklid-Therapie ist die Knochenmarksschädigung, diese kann aber durch autologe Knochenmark-Transplantation oder Stammzell-Support verbessert werden.
- Da Radionuklid-Therapien bei der Behandlung von Schilddrüsen-Krebs international etabliert sind, gibt es bereits weltweit nuklear-medizinische Therapiestationen in spezialisierten Zentren, in denen eine erfindungsgemäß beschriebene Therapie durchführbar ist.
- Neben der Therapie mit I-131 sind insbesondere auch α-Strahler (z.B. At-211) bei verschiedenen Tumorerkrankungen einsetzbar, die eine sehr hohe lokale Toxizität besitzt.

**Literatur:**
- Anderson WF: Human gene therapy. Nature 392 (6679 Suppl): 25-30, 1998.
- Bergelson JM, Cunningham JA, Drouguett G et al: Isolation of a common receptor for coxsackie B viruses and adenoviruses 2 and 5. Science 275: 1320-1323, 1997.
- Dai G et al: Cloning and charakterization of the thyroid iodide transporter. Nature 379: 458-460, London 1996.
- He TC, Zhou S, Da Costa LT, Yu J, Kinzler K.W, Vogelstein B: A simplified system for generating recombinant adenoviruses. Proc Natl Acad Sci USA 95 (5), 2509-5514, 1998.
- Kurane S, Krauss JC, Watari E, Kannagi R, Chang AE, Kudoh S: Targeted gene transfer for adenocarcinoma using a combination of tumor-specific antibody and tissue-specific promoter. Jpn J Cancer Res 89(11):1212-9, 1998.
- Malthiery Y, Lissitzky S: Primary structure of human thyroglubulin deduced from the sequence of ist 8448-base complementary DNA. Eur J Biochem 165 (3), 491-498, 1987.
- Martin F, Neil S, Kupsch J, Maurice M, Cosset F, Collins M: Retrovirus targeting by tropism restriction to melanoma cells. J Virol 73(8);6923-9; 1999.
- Metcalf KS et al: Culture of ascitic ovarian cancer cells as a clinically-relevant ex vivo model for the massessment of biological therapies. J Eur Gynaecol Oncolo 19: 113-119, 1998.
- Murphy GP et al: Measurement of serum prostate-specific membrane antigen, a new prognostic marker for prostate cancer. J Urology 51: 89-97, 1998..
- Schneider J et al: Overlapping peptides of melanocyte differentiation antigen Melan-A/MART-1 recognized by autologous cytolytic T-lymphocytes in assiciation with HLA-B45.1 and HLA-A2.1. J Cancer 75: 451-458,1998.
- Shimura et al: Iodide Uptake and experimental ¹³¹I Therapy in transplanted undifferetiated thyroid cancer cells expressing the Na+/I-symporter gene. Endocrinology 138: 4493-4496, 1997
- Short MP, Choi BC, Lee JK, Malick A, Breakefield XO, Martuza RL: Gene delivery to glioma cells in rat brain by grafting of a retrovirus packaging cell line. J Neurosci Res 27 (3): 427-39, 1990.
- Slamon DJ: Proto-oncogenes and human cancers. In: New England J Med 317: 955-957, 1987.
- Smanik PA, Liu Q, Furminger TL, Ryu K, Xing S, Mazzaferri EL, Jhiang SM: Cloning of the human sodium iodide symporter. Biochem Biophys Res Commun 226(2):339-45, 1996.
- Strauss M, Barranger JA: Concepts in gene therapy. Walter de Gruyter, Berlin New York, 1997.
- Tarhovsky YS, Ivanitsky GR: Liposomes in gene therapy. Structural polymorphism of lipids and effectiveness of gene delivery. Biochemistry 63 (6): 607-18, 1998.
- Weiss SJ, Philp NJ, Grollman EF: Iodide transport in a continuous line of cultured cells from rat thyroid. Endocrinology 1984;114(4):1090-8.

## Patentansprüche

1. Vektorkonstrukt, umfassend Vektor-DNA einschließlich regulatorischer Sequenzen sowie das NIS-Gen, das für den Natrium/Iodidsymporter kodiert, und das TPO-Gen, das für die thyreoidale Peroxidase kodiert.

2. Vektorkonstrukt nach Anspruch 1, **dadurch gekennzeichnet, dass** es zusätzlich das TG-Gen, das für das humane Thyreoglobulin kodiert, oder eine für ein physiologisch aktives Fragment davon kodierende Nukleinsäuresequenz, enthält.

3. Vektorkonstrukt nach einem der vorhergehenden Ansprüche, **dadurch gekennzeichnet, dass** sie den SV40-PE, den CMV- oder einen Gewebe spezifischen Promotor enthalten.

4. Vektorkonstrukt nach Anspruch 3, **dadurch gekennzeichnet, dass** der Gewebe spezifische Promotor der Promotor eines Gens eines Tumor spezifischen Proteins, eines Tumor spezifischen Enzyms, eines Tumor spezifischen Rezeptors, eines Tumor spezifischen Membran-Bestandteils oder eines Tumor spezifischen Tumor-Markers ist.

5. Vektorkonstrukt nach einem der vorhergehenden Ansprüche, **dadurch gekennzeichnet, dass** es zusätzlich einen Replikationsursprung und/oder ein Markergen, z.B. ein Antibiotikum-Resistenzgen, und/oder ein Polyadenylierungssignal enthält.

6. Vektorkonstrukt nach einem der vorhergehenden Ansprüche, das zusätzlich eine multiple Klonierungsstelle (multiple cloning site (MCS)), vorzugsweise in einer Position in 5'-Richtung von dem NIS-Gen, enthält.

7. Vektorkonstrukt nach einem der Ansprüche 1 bis 5, das zusätzlich eine multiple Klonierungsstelle (multiple cloning site (MCS)), vorzugsweise in einer Position in 3'-Richtung von dem NIS-Gen, enthält.

8. Vektorkonstrukt nach einem der vorhergehenden Ansprüche, **dadurch gekennzeichnet, dass** es in einem Liposom verpackt vorliegt, wobei die Liposomen Membran ständige Antikörper, insbesondere monoklonale Antikörper, oder andere Proteine, insbesondere Rezeptor-Liganden, gegen Tumoroberflächen-Strukturen aufweisen können.

9. Vektorkonstrukt nach einem der Ansprüche 1 bis 7, **dadurch gekennzeichnet, dass** es in rekombinante adenovirale, retrovirale oder andere human- oder tier-pathogene Viren verpackt vorliegt, wobei diese an ihrer Oberfläche natürliche oder künstliche Gewebe spezifische, Membran ständige Proteine (z.B. Fiber-Proteine) oder Rezeptor-Liganden gegen Tumoroberflächen-Strukturen aufweisen können.

10. Vektorkonstrukt nach Anspruch 9, **dadurch gekennzeichnet, dass** es replikationsdefizient ist.

11. Vektorkonstrukt nach einem der vorhergehenden Ansprüche für die Verwendung in der Human- oder Veterinärmedizin.

12. Verwendung des Vektorkonstrukts nach einem der Ansprüche 1 bis 10 zur Herstellung eines Medikaments/Diagnostikums zur Behandlung/Diagnose von Tumorerkrankungen, wobei die Behandlung vor einer oder gleichzeitig mit einer Radionuklid-Therapie, insbesondere mit 131-Iod oder 211-Astat, erfolgt.

13. Verwendung von zwei oder von mehreren Vektorkonstrukten zur Herstellung eines Medikaments/Diagnostikums zur Behandlung/Diagnose von Tumorerkrankungen, wobei die Behandlung vor einer oder gleichzeitig mit einer Radionuklid-Therapie, insbesondere mit ¹³¹Iod oder ²¹¹Astat, erfolgt, **dadurch gekennzeichnet, dass** die zwei oder mehreren Vektorkonstrukte jeweils Vektor-DNA einschließlich regulatorischer Sequenzen sowie, in verschiedenen Konstrukten, das NIS-Gen, das für den Natrium/Iodidsymporter kodiert, das TPO-Gen, das für die thyreoidale Peroxidase kodiert, und ggf.. das TG-Gen, das für das humane Thyreoglobulin oder eines seiner Untereinheiten kodiert, enthalten, wobei die zwei oder mehreren Vektorkonstrukte ggf. in Liposomen, wobei die Liposomen Membran ständige Antikörper, insbesondere monoklonale Antikörper, oder andere Proteine, insbesondere Rezeptor-Liganden, gegen Tumoroberflächen-Strukturen aufweisen können, in adenovirale, retrovirale oder andere human- oder tier-pathogene virale Vektoren, wobei das Konstrukt an seiner Oberfläche natürliche oder künstliche Gewebe spezifische, Membran ständige Proteine (z.B. Fiber-Proteine) oder Rezeptor-Liganden gegen Tumoroberflächen-Strukturen aufweisen kann, oder in beiden verpackt vorliegen.

14. Verwendung nach Anspruch 12 oder 13, wobei die Radionuklid-Therapie mit Astat-211 (als At- oder als anionische Verbindung, insbesondere als AtO⁻, AtO₃⁻, AtO₄⁻, AtO₆⁵⁻), mit Rhenium-188 bzw. Rhenium-186 (als anionische Verbindung) oder mit Yttrium-90 (als anionische Verbindung) erfolgt.

15. Verwendung nach einem der Ansprüche 12 bis 14, **dadurch gekennzeichnet, dass** das Medikament/Diagnostikum intravenös, intraperitoneal, intrathekal, intracraniell, intrathorakal, endobronchial, endolymphatisch, intraarteriell oder intratumoral appliziert wird.

16. Verwendung nach einem der Ansprüche 12 bis 15, **dadurch gekennzeichnet, dass** die Tumorerkrankung ein entdifferenziertes und medulläres Schilddrüsen-Karzinom, ein Magen- oder Darmtumor, ein Leber-, Hirn-, Knochen-, Muskel-, Nieren-, Blasen-, Mundboden-, Uterus-, Lungen- oder Keimdrüsen-Tumor, ein Haut-Tumor oder ein Tumor der exokrinen und endokrinen Drüsen ist.

17. Liposom, **dadurch gekennzeichnet, daß** es eines oder mehrere der Vektorkonstrukte nach einem der Ansprüche 1 bis 9 enthält.

18. Liposom nach Anspruch 17, **dadurch gekennzeichnet, dass** in der Membran des Liposoms Antikörper, insbesondere monoklonale Antikörper, oder Proteine, insbesondere Rezeptor-Liganden, gegen Tumorzell-Oberflächenstrukturen vorliegen.

19. Rekombinantes adenovirales, retrovirales oder anderes human- oder tier-pathogenes Virus, **dadurch gekennzeichnet, dass** es eines oder mehrere der Vektorkonstrukte nach einem der Ansprüche 1 bis 9 enthält.

## Claims

1. A vector construct, comprising vector DNA including regulatory sequences as well as the NIS gene encoding the sodium/iodide symporter and the TPO gene encoding the thyroid peroxidase.

2. The vector construct according to claim 1, **characterised in that** it further comprises the TG gene encoding the human thyreoglobulin or a nucleic acid sequence encoding a physiologically active fragment of human thyreoglobulin.

3. The vector construct according to any of the preceding claims, **characterised in that** it comprises the SV40-PE, CMV, or a tissue-specific promoter.

4. The vector construct according to claim 3, **characterised in that** the tissue-specific promoter is the promoter of a gene of a tumour-specific protein, of a tumour-specific enzyme, of a tumour-specific receptor, of a tumour-specific membrane component, or of a tumour-specific tumour marker.

5. The vector construct according to any of the preceding claims, **characterised in that** it further comprises an origin of replication and/or a marker gene, e.g., an antibiotic resistance gene, and/or a polyadenylation signal.

6. The vector construct according to any of the preceding claims, further comprising a multiple cloning site (MCS), preferably in a position in 5' direction of the NIS gene.

7. The vector construct according to any of claims 1 to 5, further comprising a multiple cloning site (MCS), preferably in a position in 3' direction of the NIS gene.

8. The vector construct according to any of the preceding claims, **characterised in that** it is included in a liposome, wherein the liposomes may exhibit membrane-bound antibodies, in particular monoclonal antibodies, or other proteins, in particular receptor ligands, specific for tumour surface structures.

9. The vector construct according to any of claims 1 to 7, **characterised in that** it is included in recombinant adenoviral, retroviral or other viruses of human or animal pathogenicity, wherein the viruses may exhibit on their surface natural or artificial tissue-specific membrane-bound proteins (e.g., fiber proteins) or receptor ligands specific for tumour surface structures.

10. The vector construct according to claim 9, **characterised in that** is replication-deficient.

11. The vector construct according to any of the preceding claims for the use in human or veterinary medicine.

12. Use of the vector construct according to any of claims 1 to 10 for the manufacture of a medicament/diagnostic agent for the treatment/diagnosis of tumour diseases, wherein the treatment is carried out prior to or simultaneously with a radionuclide therapy, in particular with iodine-131 or astate-211.

13. Use of two or more vector constructs for the manufacture of a medicament/diagnostic agent for the treatment/diagnosis of tumour diseases, wherein the treatment is carried out prior to or simultaneously with a radionuclide therapy, in particular with iodine-131 or astate-211, **characterised in that** the two or more vector constructs each contain vector DNA including regulatory sequences and, in different constructs, the NIS gene encoding the sodium/iodide symporter, the TPO gene encoding the thyroid peroxidase, and optionally the TG gene encoding the human thyreoglobulin or any of its sub-units, wherein the two or more vector constructs are optionally included in liposomes, wherein the liposomes may exhibit membrane-bound antibodies, in particular monoclonal antibodies, or other proteins, in particular receptor ligands, specific for tumour surface structures, or are included in adenoviral, retroviral, or other viral vectors of human or animal pathogenicity, wherein the construct may exhibit on its surface natural or artificial tissue-specific, membrane-bound proteins (e.g., fiber proteins), or receptor ligands specific for tumour surface structures, or are included in both.

14. Use according to claim 12 or 13, wherein the radionuclide therapy is performed with At-211 (as At- or as anionic compound, in particular as AtO⁻, AtO₃⁻, AtO₄⁻, AtO₆⁵⁻), with rhenium-188 and rhenium-186 (as anionic compound) or with yttrium-90 (as anionic compound).

15. Use according to any of claims 12 to 14, **characterised in that** the medicament/diagnostic agent is administered via the intravenous, intraperitoneal, intrathecal, intracranial, intrathoracal, endobronchial, endolymphatic, intraarterial, or intratumoral route.

16. Use according to any of claims 12 to 15, **characterised in that** the tumour disease is a dedifferentiated and medullar thyroid carcinoma, a stomach or intestine tumour, a liver, brain, bone, muscle, kidney, bladder, mylohyoid, uterus, lung, or gonadal tumour, a skin tumour, or a tumour of the exocrine and endocrine glands.

17. A liposome, **characterised in that** it comprises one or more of the vector constructs according to any of claims 1 to 9.

18. The liposome according to claim 17, **characterised in that** it harbours in its membrane antibodies, in particular monoclonal antibodies, or proteins, in particular receptor ligands, specific for tumour cell surface structures.

19. A recombinant adenoviral, retroviral or other virus of human or animal pathogenicity, **characterised in that** it comprises one or more of the vector constructs according to any of claims 1 to 9.

## Revendications

1. Construction de vecteur, comprenant un ADN vecteur incluant des séquences de régulation ainsi que le gène NIS, qui code pour le symporteur Sodium/Iodure, et le gène TPO, qui code pour la peroxydase thyroïdienne.

2. Construction de vecteur selon la revendication 1, **caractérisée en ce qu'**elle contient en outre le gène TG, qui code pour la thyroglobuline humaine, ou une séquence d'acide nucléique codant pour un fragment physiologiquement actif de celle-ci.

3. Construction de vecteur selon l'une quelconque des revendications précédentes, **caractérisée en ce qu'**elle contient le SV40-PE, le CMV ou un promoteur spécifique de tissu.

4. Construction de vecteur selon la revendication 3, **caractérisée en ce que** le promoteur spécifique d'un tissu est le promoteur d'un gène d'une protéine spécifique d'une tumeur, d'une enzyme spécifique d'une tumeur, d'un récepteur spécifique d'une tumeur, d'un composant membranaire spécifique d'une tumeur ou d'un marqueur tumoral spécifique d'une tumeur.

5. Construction de vecteur selon l'une quelconque des revendications précédentes, **caractérisée en ce qu'**elle contient en outre une origine de réplication et/ou un gène marqueur, par exemple un gène de résistance aux antibiotiques et/ou un signal de polyadénylation.

6. Construction de vecteur selon l'une quelconque des revendications précédentes, qui contient en outre un site de clonage multiple (multiple cloning site, MCS), de préférence à une position en direction 5' du gène NIS.

7. Construction de vecteur selon l'une quelconque des revendications 1 à 5, qui contient en outre un site de clonage multiple (multiple cloning site, MCS), de préférence à une position en direction 3' du gène NIS.

8. Construction de vecteur selon l'une quelconque des revendications précédentes, **caractérisée en ce qu'**elle est enveloppée dans un liposome, la membrane desdits liposomes pouvant présenter des anticorps permanents, en particulier des anticorps monoclonaux, ou d'autres protéines, en particulier des ligands de récepteurs, dirigés contre des structures de surfaces tumorales.

9. Construction de vecteur selon l'une quelconque des revendications 1 à 7, **caractérisée en ce qu'**elle est enveloppée dans un adénovirus, un rétrovirus ou d'autres virus recombinants pathogènes pour l'homme ou les animaux, lesdits virus pouvant présenter à leur surface des protéines membranaires permanentes, spécifiques de tissus, naturelles ou artificielles (par exemple des protéines de fibres) ou des ligands de récepteurs dirigés contre des structures de surfaces tumorales.

10. Construction de vecteur selon la revendication 9, **caractérisée en ce qu'**elle est défective pour la réplication.

11. Construction de vecteur selon l'une quelconque des revendications précédentes destinée à être utilisée en médecine humaine ou vétérinaire.

12. Utilisation de la construction de vecteur selon l'une quelconque des revendications 1 à 10 pour la fabrication d'un médicament / d'un produit de diagnostic pour le traitement/le diagnostic de maladies tumorales, ledit traitement étant effectué avant ou simultanément à un traitement par un nucléide radioactif, en particulier avec de l'iode 131 ou de l'astate 211.

13. Utilisation de deux ou plusieurs constructions de vecteur pour la fabrication d'un médicament / d'un produit de diagnostic pour le traitement / le diagnostic de maladies tumorales, ledit traitement étant effectué avant ou simultanément à un traitement par un nucléide radioactif, en particulier avec de l'iode¹³¹ ou de l'astate²¹¹, **caractérisé en ce que** les deux ou plusieurs constructions de vecteurs contiennent chacune un ADN vecteur incluant des séquences de régulation ainsi que, dans différentes constructions de vecteurs, le gène NIS, qui code pour le symporteur Sodium/Iodure, le gène TPO, qui code pour la peroxydase thyroïdienne, et le cas échéant le gène TG, qui code pour la thyroglobuline humaine ou l'une de ses sous-unités, lesdites deux ou plusieurs constructions de vecteurs étant enveloppées, le cas échéant dans des liposomes, la membrane des liposomes pouvant présenter des anticorps permanents, en particulier des anticorps monoclonaux, ou d'autres protéines, en particulier des ligands de récepteurs, dirigés contre des structures de surfaces tumorales, dans des vecteurs d'adénovirus, de rétrovirus ou d'autres virus pathogènes pour l'homme ou les animaux, ladite construction pouvant présenter à sa surface des protéines membranaires permanentes, spécifiques de tissus, naturelles ou artificielles (par exemple des protéines de fibres) ou des ligands de récepteurs dirigés contre des structures de surfaces tumorales, ou dans les deux.

14. Utilisation selon la revendication 12 ou 13, dans laquelle le traitement par les nucléides radioactifs est effectué avec l'astate 211 (sous forme d'At- ou de composés anioniques, en particulier sous forme d'AtO⁻, d'AtO₃⁻, d'AtO₄⁻, d'AtO₆⁵⁻), avec le rhénium 188 ou le rhénium 186 (sous forme de composés anioniques) ou avec l'yttrium 90 (sous forme de composés anioniques).

15. Utilisation selon l'une quelconque des revendications 12 à 14, **caractérisée en ce que** le médicament / produit de diagnostic est administré par voie intraveineuse, intra-péritonéale, intrathécale, intracrânienne, intra-thoracique, endobronchique, endolymphatique, intra-artérielle ou intra-tumorale.

16. Utilisation selon l'une quelconque des revendications 12 à 15, **caractérisée en ce que** la maladie tumorale est un carcinome dédifférencié et médullaire de la thyroïde, une tumeur de l'estomac ou de l'intestin, une tumeur du foie, du cerveau, des os, des muscles, des reins, de la vessie, du plancher buccal, de l'utérus, des poumons ou des gonades, une tumeur de la peau ou une tumeur des glandes exocrines et endocrines.

17. Liposome **caractérisé en ce qu'**il contient une ou plusieurs constructions de vecteurs selon l'une quelconque des revendications 1 à 9.

18. Liposome selon la revendication 17, **caractérisé en ce que** dans la membrane du liposome se trouvent des anticorps, en particulier des anticorps monoclonaux, ou des protéines, en particulier des ligands de récepteurs dirigés contre les structures de surface des cellules tumorales.

19. Adénovirus, rétrovirus ou autre virus recombinant pathogène pour l'homme et les animaux, **caractérisé en ce qu'**il contient une ou plusieurs constructions de vecteurs selon l'une quelconque des revendications 1 à 9.
